# EUROPEAN PATENT APPLICATION

(11) **EP 1 306 047 A2**
(43) Date of publication of application: **02.05.2003**
(21) Application number: 02256282.1
(22) Date of filing: 11.09.2002
(51) Int. Cl.: A61B 1/00

(54) **Endoscope**

(30) Priority: 29.10.2001 JP 2001331087
(71) Applicant: MACHIDA ENDOSCOPE CO., LTD, Bunkyo-ku, Tokyo (JP)
(72) Inventor: Miyagi, Kunihiko, c/o Machida Endoscope Co., Ltd., Tokyo (JP); Ito, Haruo, c/o Machida Endoscope Co., Ltd., Tokyo (JP); Nishimura, M., c/o Machida Endoscope Co., Ltd., Tokyo (JP); Inoue, Masahiro, c/o Machida Endoscope Co., Ltd., Tokyo (JP)
(74) Representative: Hallybone, Huw George

(57) **Abstract**

A light source device 20 of an endoscope is provided with a light guide connector 24 for inserting a light guide plug 15. A ball lens 30 (light intensity distribution transforming element) is received in an end portion on a condenser lens 23 side of the light guide connector 24. The ball lens 30 transforms the intensity distribution of a converging flux of light coming from the condenser lens 23 such that the intensity distribution is lowered at the central area and raised at the peripheral area. A ball lens 31 (light arresting element) is received in an end portion of the light guide plug 15. The ball lens 31 arrests the light coming from the ball lens 30 and makes incidence of such arrested light to an incident end face of the light guide 16. Owing to this feature, the inserting operation of the light guide plug can be performed with ease and without a need of strict accuracy.

## Description

### BACKGROUND OF THE INVENTION

This invention relates to an endoscope device in which an endoscope and a light source device for supplying an illumination light to this endoscope are separatably connected together.

In general, an endoscope and a light source device are separately formed. The endoscope is provided with a light guide plug in which an incident end portion of a light guide is received. At the time of use, this light guide plug is inserted for connection into a light guide connector of the light source device. And the light source device is turned on. Then, within the light source device, an illumination light coming from a light source is converged through a condenser lens and made incident to an incident end face of the light guide. This incident light is guided by the light guide and emitted from an outgoing end of the light guide facing a tip of an insertion portion of the endoscope so as to illuminate an object to be observed.

Recently, endoscopes have been demanded to be made very fine at the insertion portion. In such endoscopes, the number of optical fibers which can be received as a light guide is limited and the incident end face is very small in diameter. In order to make the light, which was converged through the condenser lens, incident to this very small incident end face, not only the condensing accuracy of the condenser lens but also the positioning accuracy of both the condenser lens and the light guide and in addition, the connecting accuracy between the light guide connector and the light guide plug are required to be set to a high level. Accordingly, the manufacturing cost is increased. Moreover, the user side is compelled to do work for maintaining such accuracy. Thus, convenient efficiency is lowered.

### SUMMARY OF THE INVENTION

The present invention has been proposed in order to solve the above-mentioned problems.

A main features of the present invention resides in an endoscope device comprising (A) a light source device including a light source, a condenser lens for converging an illumination light coming from the light source and a light guide connector; and (B) an endoscope including a light guide plug which can be inserted into and removed from the light guide connector and a light guide whose incident end is received in the light guide plug; the light source device being provided with a light intensity distribution transforming element and the light guide plug of the endoscope being provided with a light arresting element. The light intensity distribution transforming element transforms intensity distribution of a converging flux of light coming from the condenser lens such that the intensity distribution is lowered at a central area and raised at a peripheral area. The light arresting element arrests the light coming from the light intensity distribution transforming element and makes incidence of such arrested light to an incident end face of the light guide in a state that the light guide plug is inserted in the light guide connector. Owing to the foregoing feature, even if the light guide plug is not correctly positioned with respect to the light guide connector of the light source device, a light having a sufficient intensity can be made incident to the light guide. Accordingly, the user can perform the inserting operation of the light guide plug with ease and the convenient efficiency of the endoscope device can be enhanced extensively.

It is preferred that the light guide connector includes a plug insertion hole for the light guide plug and a transforming element receiving hole continuous with the condenser lens side of the plug insertion hole, and the light intensity distribution transforming element is received in the transforming element receiving hole. Owing to the foregoing feature, the light intensity distribution transforming element and the light arresting element can surely be placed in proximate and facing relation with each other and the illumination light coming from the light source can surely be made incident to the light guide.

Preferably, the light intensity distribution transforming element and the light arresting element are lenses, such as ball lenses or the like, which are lower in condensing degree of light than the condenser lens. Owing to the foregoing feature, the manufacturing cost can be decreased.

### BRIEF DESCRIPTION OF THE DRAWINGS

FIG. 1 is a diagram schematically showing a general construction of an endoscope device according to one embodiment of the present invention;
FIG. 2 is a sectional view showing an essential part of the above endoscope device;
FIG. 3 is a view showing an optical system of the endoscope device and an optical path passing therethrough; and
FIG. 4 is a graph in which an intensity distribution of a flux of light at the focus of a ball lens for transforming a light intensity distribution is indicated by a solid line and an intensity distribution at the focus of a condenser lens in absence of the ball lens is indicated by an imaginary line.

### DETAILED DESCRIPTION OF THE INVENTION

One embodiment of the present invention will now be described with reference to the accompanying drawings.

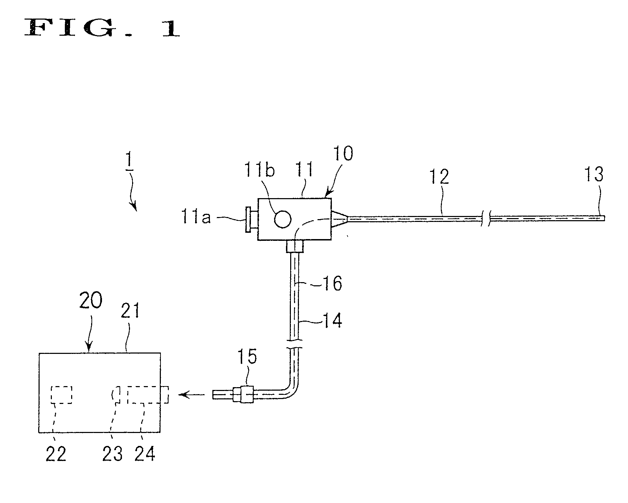

FIG. 1 shows an endoscope device 1. The endoscope device 1 includes an endoscope 10 and a light source device 20 for supplying an illumination light to the endoscope 10.

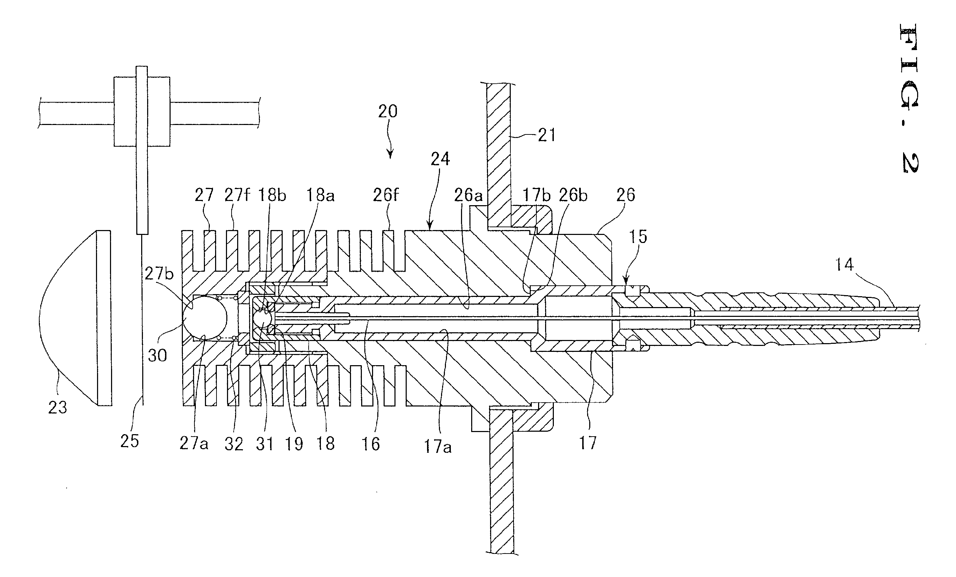

The light source device 20 includes a housing 21. Received in the housing 21 are a light source 22, a condenser lens 23 and a light guide connector 24. Those components 22 through 24 received in the housing 21 are linearly arranged along an optical axis of the illumination light coming from the light source 22. A diaphragm 25 (see FIG. 2) is provided between the condenser lens 23 and the light guide connector 24. Since the light guide connector 24 includes an essential part of the present invention, it will be described in more detail later.

The endoscope 10 includes a main body portion 11, a flexible insertion portion 12 extending from the main body portion 11 and a tip component portion 13 disposed at a tip of this insertion portion 12. The insertion portion 12 and the tip component portion 13 are widthwise finely designed so that they can be inserted into a blood vessel, for example.

The main body portion 11 is provided with an observing ocular portion 11a and a control knob 11b for bending the insertion portion. A distal end of a light guide cable 14 is connected to the main body portion 11. A light guide plug 15 is attached to a basal end of the light guide cable 14. This light guide plug 15 is removably connected to the light guide connector 24. Since the light guide plug 15 includes an essential part of the present invention, it will be described in more detail later.

The light guide plug 15, the light guide cable 14, the main body 11, the insertion portion 12 and the tip component portion 13 receive therein a light guide 16 which is composed of a flux of optical fibers (or single optical fiber). The light guide 16 is adapted to introduce the illumination light coming from the light source device 20 to an illumination window (not shown) formed in the tip component portion 13. The number of optical fibers, which compose the light guide 16, limited to small in order to cope with the insertion portion 12 and the tip component portion 13 which are widthwise finely designed.

Accordingly, an incident end face (basal end face) of the light guide 16 is size-wise finely designed. In order to surely make incident of the illumination light to the finely designed incident end face, as shown in FIG. 2, a pair of ball lenses 30, 31 (optical elements) is provided on an illumination optical path between the condenser lens 23 and the light guide 16. The condensing degree of the ball lenses 30, 31 is lower than that of the condenser lens 23. The large ball lens 30 (light intensity distribution transforming element) on the side of the condenser lens 23 is disposed at the light guide connector 24 and the small ball lens 31 (light arresting element) on the side of the light guide 16 is disposed at the light guide plug 15.

The light guide connector 24 includes a connector main body 26 attached to the housing 21 and a lens receiving member 27 threadingly engaged with the condenser lens 23 side of the connector body 26. Those components 26, 27 are formed of material having a good thermal conductivity such as, for example, brass. Heat radiation fins 26f, 27f are provided on outer peripheries of the components 26, 27. This makes it easy to release heat caused by the illumination light.

A plug insertion hole 26a for the light guide plug 15 is formed in the connector main body 26. A lens receiving hole 27a (transforming element receiving hole), which is coaxially continuous with the plug insertion hole 26a, is formed in the lens receiving member 27. The light intensity distribution transforming ball lens 30 is received in this lens receiving hole 27a.

The inside diameter of the lens receiving hole 27a is dimensioned large enough to allow the thermal expansion of the ball lens 30. In the lens receiving hole 27a, a lens retaining potion 27b (transforming element retaining portion) is provided at an inner peripheral edge on the condenser lens 23 side, and a coiled spring 32 (transforming element biasing means) is received in an area more on the plug insertion hole 26a side than the ball lens 30. This coiled springs 32 urges the ball lens 30 against the lens retaining portion 27b. By this, the ball lens 30 is fixed without swaying.

The light guide plug 15 includes a plug main body 17 and a plug cap 18 threadingly engaged with an end portion of this plug main body 17. A guide receiving hole 17a is formed in the plug main body 17. An incident end portion of the light guide 16 is received in this guide receiving hole 17a. A lens receiving hole 18a (arresting element receiving hole), which is coaxially continuous with the guide receiving hole 17a, is formed in the plug cap 18. The optical arresting ball lens 31 is received in this lens receiving hole 18a. An incident end face of the light guide 16 is correctly positioned on this ball lens 31.

The inside diameter of the lens receiving hole 18a is dimensioned large enough to allow the thermal expansion of the ball lens 31. A lens retaining portion 18b (arresting element retaining portion) is disposed at an inner periphery on the tip side of the lens receiving hole 18a. A resin-made resilient ring 19 (arresting element biasing means) is sandwiched between the plug cap 18 and a distal end face of the plug main body 17. This resilient ring 19 urges the ball lens 31 against the lens retaining portion 18b. By this, the ball lens 31 is fixed without swaying.

A step 17b is formed on an outer periphery of the plug main body 17 of the light guide plug 15. Abutment of the step portion 17b with a step portion 26b formed on an inner periphery of the connector main body 26 determines a constant insertion depth of the light guide plug 15 into the light guide connector 24. By this, a certain degree of positioning accuracy of the light guide plug 15 with respect to the light guide connector 24 is obtained. In that condition, the pair of ball lenses 30, 31 are faced with each other with a predetermined distance held therebetween.

Operation will now be described.

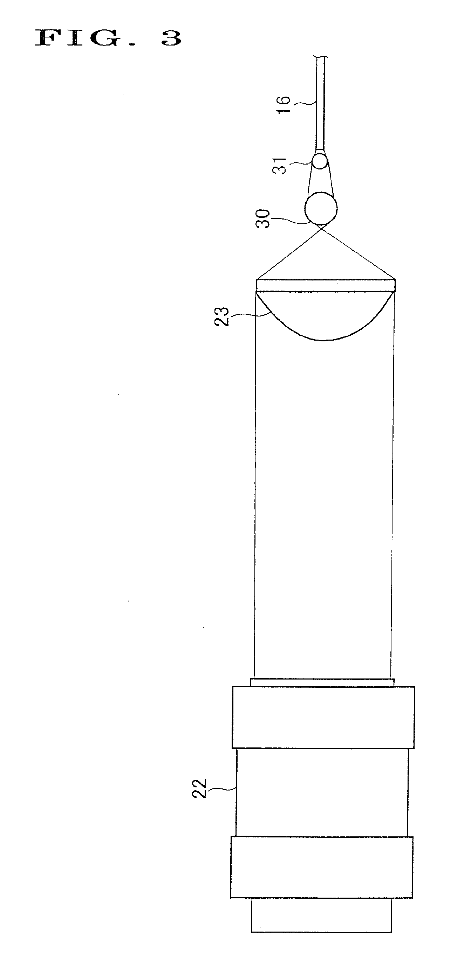

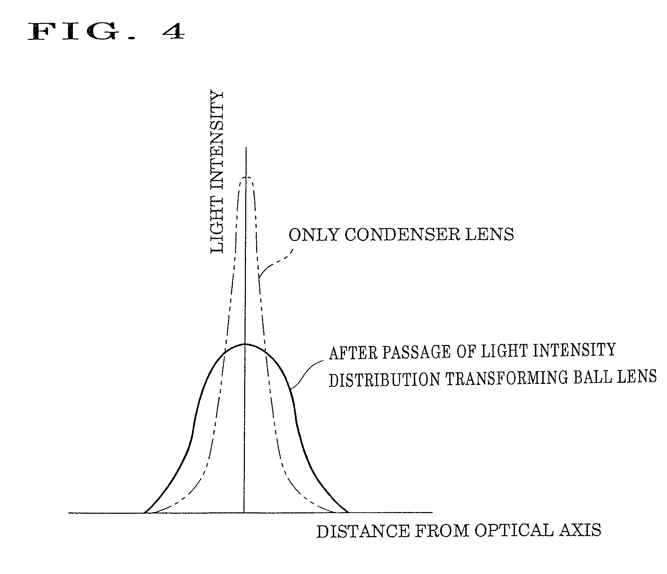

As shown in FIG. 3, the light source 22 emits an illumination light towards the condenser lens 23 generally in parallel relation. The condenser lens 23 converges this light into a converged flux of light. After passing through the condenser lens 23, the light is reduced in diameter of the flux of light as it progresses. At the same time, the light is increased in intensity at a central area (nearby area of the optical axis) of the flux of light and decreased in intensity at a peripheral area. Presuming that there is no provision of the ball lens 30 of the light guide connector 24 at the tip of the condenser lens 23, the central area of the flux of light reaches the peak in intensity at the focus of the condenser lens 23 as indicated by an imaginary line of FIG. 4. And the intensity of light is rapidly decreased, as it is displaced, even if slightly, from the center.

Actually, the converging flux of light is made incident to the ball lens 30 of the light guide connector 24 in the vicinity of the focus of the condenser lens 23. This ball lens 30 is lower in condensing degree of light than the condenser lens 23. Because of this reason, after passing through the ball lens 30, the flux of light is transformed such that the intensity distribution is lowered at the central area and raised at the peripheral area as indicated by a solid line of FIG. 4. The diameter of the flux of light is maintained generally in the same dimension as the one when it is contracted.

Thereafter, the light travels towards the ball lens 31 of the light guide plug 15. Since this ball lens 31 is sufficiently large in diameter compared with the contracted diameter of the flux of light, it can surely arrest generally the entire light even if the position is slightly displaced with respect to the ball lens 30 of the light guide connector 24. That is, it can surely arrest both the central area and the peripheral area of the flux of light.

The ball lens 31 converges the arrested flux of light towards the light guide 16. At that time, even if the center, i.e., optical axis, of the flux of light is displaced from the axis of the incident end portion of the light guide 16 and only the peripheral area of the flux of light is made incident to the light guide 16, the intensity of the incident light is sufficiently large. This light is guided by light guide 16 and emitted through an illumination window formed in the tip component portion 13 so as to hit an object to be observed. By this, it can illuminate the object brightly.

As apparent from the foregoing, since there is no inconvenience for the light to illuminate even if the optical axis is slightly displaced from the axis of the light guide 16, strict positioning accuracy of the light guide connector 24 with respect to the light guide plug 15 is not required. As a result, the user can easily perform the insertion operation of the light guide plug 15, and convenient efficiency of the endoscope device 1 can be enhanced extensively.

Since the ball lens 30 of the light guide connector 24 acts to defocus, uneven intensity of light at a light emitting surface of the light source 22 can be uniformed. Moreover, the shadow of the diaphragm 25 can be removed.

Since the ball lens 31 of the light guide plug 15 acts to increase the maximum incident angle of the light directing to the light guide 16, the maximum outgoing angle from the illumination window is also increased and thus, the range of area for the light to illuminate can be increased.

By using the ball lenses 30, 31 as the light intensity distribution transforming element and the light arresting element, the manufacturing cost can be reduced.

Since the plug cap 18 is threadingly engaged with the plug main body 15, it can easily be removed. By this, the light distribution characteristic and other characteristics of the illumination can be adjusted optionally by replacing the ball lens 31 with one which has a wide variety of specifications..

The present invention is not limited to the above embodiment, and many changes and modifications can be made. For example, instead of the ball lenses 30, 31, convex lenses having a small condensing degree of light may be used as the light intensity distribution transforming element and the light arresting element.

It is also accepted that the light source and the condenser lens are integrally formed as a light source unit.

## Claims

1. An endoscope device(1) comprising:
(A) a light source device(20) including a light source(22), a condenser lens(23) for converging an illumination light coming from said light source (22) and a light guide connector(24); and
(B) an endoscope(10) including a light guide plug(15) which can be inserted into and removed from said light guide connector(24) and a light guide(16) whose incident end is received in said light guide plug(15);
**CHARACTERIZED in that** said light source device(20) being provided with a light intensity distribution transforming element(30) for transforming intensity distribution of a converging flux of light coming from said condenser lens(23) such that the intensity distribution is lowered at a central area and raised at a peripheral area; and
said light guide plug(15) of said endoscope(10) being provided with a light arresting element(31) for arresting the light coming from said light intensity distribution transforming element(30) and making incidence of such arrested light to an incident end face of said light guide(16) in a state that said light guide plug(15) is inserted in said light guide connector(24).

2. An endoscope device(1) according to claim 1, wherein said light guide connector(24) includes a plug insertion hole(26a) for said light guide plug(15) and a transforming element receiving hole(27a) continuous with the condenser lens(23) side of said plug insertion hole(26a), and said light intensity distribution transforming element(30) is received in said transforming element receiving hole(27a).

3. An endoscope device(1) according to claim 1, wherein said light intensity distribution transforming element(30) and said light arresting element(31) are lenses which are lower in condensing degree of light than said condenser lens(23).

4. An endoscope device(1) according to claim 1, wherein said light intensity distribution transforming element(30) and said light arresting element(31) are ball lenses.
